# EUROPEAN PATENT APPLICATION

(11) **EP 4 822 016 A1**
(43) Date of publication of application: **14.10.2026**
(21) Application number: 25169151.5
(22) Date of filing: 08.04.2025
(51) Int. Cl.: C12N 5/0783, C12N 5/00

(54) **MEANS AND METHODS FOR ISOLATING T CELLS**

(71) Applicant: The Automation Partnership (Cambridge) Limited, Royston, Hertfordshire SG8 5WY (GB)
(72) Inventor: GERMEROTH, Lothar, 81679 Munich (DE)
(74) Representative: Cohausz & Florack

(57) **Abstract**

The invention relates to methods of isolating T cells, comprising contacting T cells with a selection agent that is specific for a receptor on the T cells and incubating the T cells at conditions inducing downregulation of said receptor, resulting in relase of the T cells from the selection agent. The invention also relates to compositions comprising said T cells and related medical uses.

## Description

### FIELD OF THE INVENTION

The invention relates to methods of isolating T cells, comprising contacting T cells with a selection agent that is specific for a receptor on the T cells and incubating the T cells at conditions inducing downregulation of said receptor, resulting in relase of the T cells from the selection agent. The invention also relates to compositions comprising said T cells and related medical uses.

### BACKGROUND

T cell selection and control over T cell composition is a cornerstone of all current cell manufacturing for therapeutic application. However, many selection techniques involve steps that contaminate target cells with selection particles or require addition of reagents that interfere with downstream processes. In addition, these purification approaches include multiple washing and/or media exchange steps that can be considered as cell stress factors and require considerable processing times.

There is hence a need in the art to provide improved methods for isolating T cells.

### SUMMARY OF THE INVENTION

The invention relates to a method of isolating one or more T cells, the method comprising (a) contacting a plurality of T cells with a selection agent that is specific for a receptor on one or more of the T cells, (i) wherein the selection agent is attached to a solid phase; or (ii) wherein the selection agent is not attached to a solid phase and the method further comprises attaching the selection agent to a solid phase; and (b) incubating the one or more T cells at about 19 °C to about 42°C for about 1 h to about 24 h, thereby inducing downregulation of said receptor on the T cell, resulting in relase of one or more of the plurality of the T cells from the selection agent; wherein steps (a) and (b) are conducted in absence of an oligomeric stimulatory reagent that comprises a specific binding partner for CD3 and/or a specific binding partner for CD28.

The invention also relates to a composition comprising one or more T cells obtainable by a method of isolating one or more T cells of the invention.

The invention also relates to a method of treating a disease comprising administering to a subject one or more T cells or a composition comprising said one or more T cells obtainable by a method of isolating one or more T cells of the invention.

The invention also relates to one or more T cells obtainable by a method of the invention or a composition of the invention for use in therapy.

The invention also relates to a use of one or more T cells obtainable by the method of the invention or a composition of the invention for the manufacture of a medicament.

### BRIEF DESCRIPTION OF THE DRAWINGS

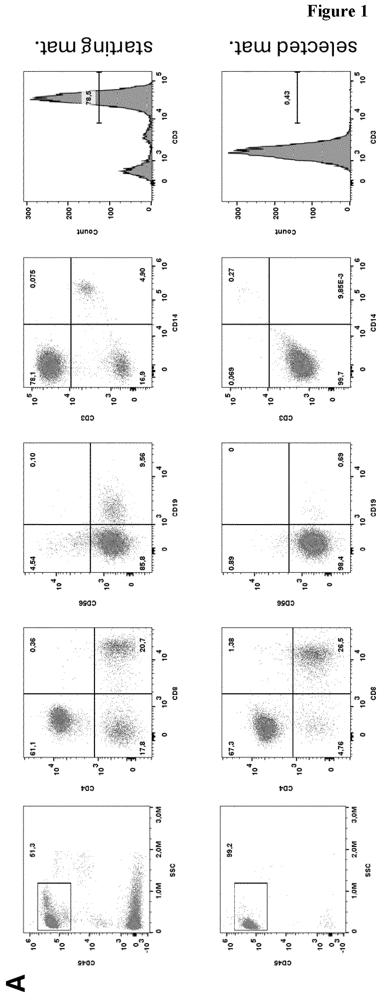

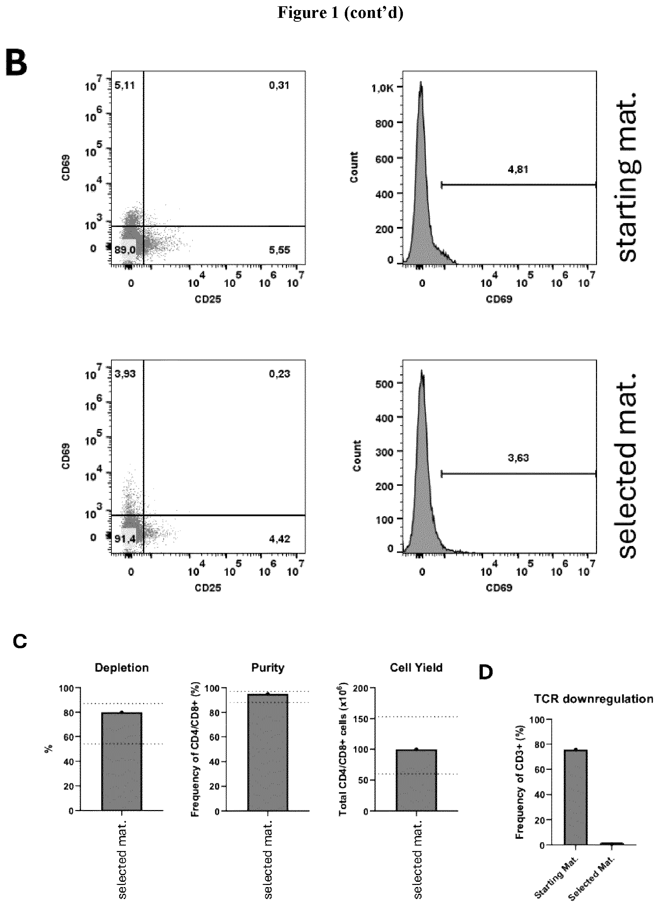

**Figure 1****: Isolation of target T cells using cell chromatography resin without addition of stimulus or elutant. A)** Representative dot plots and histograms display phenotype of cells before (starting material) and after (selected material) CD3 cell selection from PBMC fraction of a buffy coat. **B)** Representative dot plots and histograms display activation state of cells before and after selection. **C)** Graphs depict basic selection parameters such as depletion efficiency, purity, yield. Dotted lines indicate average parameter ranges when stimulation agent is added. **D)** Graph presents TCR/CD3 complex downregulation after selection as a frequency of CD3 cells.

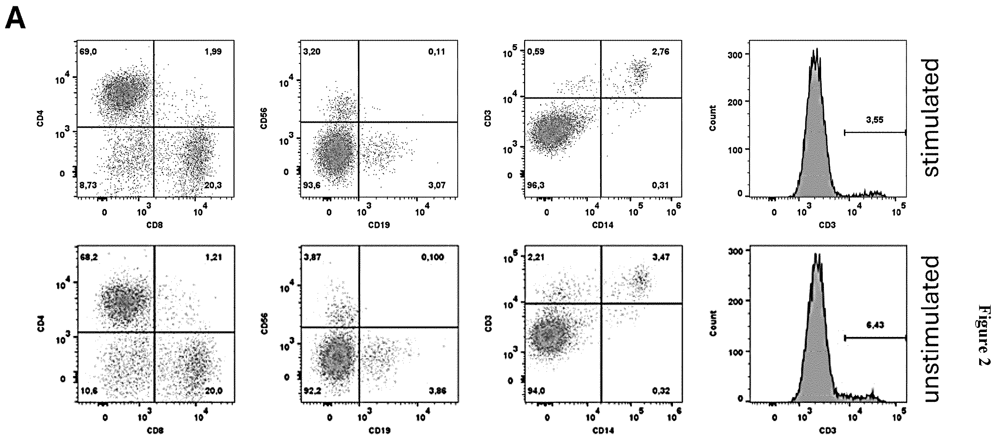

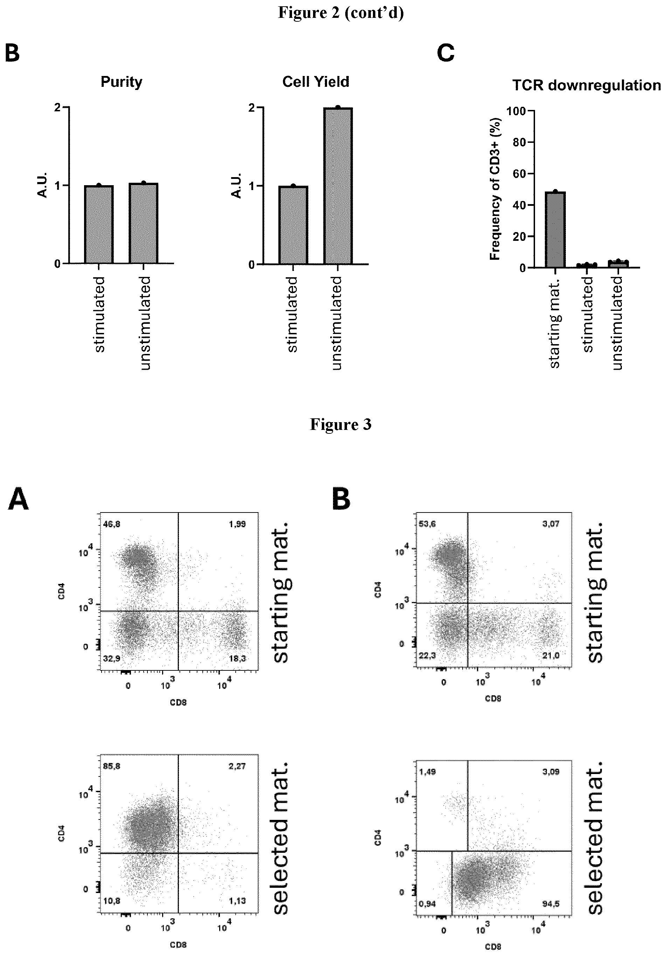

**Figure 2****: Isolation of target T cells using synthetic bead particles in solution. A)** Representative dot plots and histograms display phenotype of CD3-selected cells with (stimulated) and without (unstimulated) addition of stimulation agent. **B)** Graphs depict basic selection parameters - purity and yield. **C)** Graph presents TCR/CD3 complex downregulation after selection as a frequency of CD3 cells.
**Figure 3****: Isolation of target T cells via CD4 or CD8 co-receptor using cell chromatography resin without addition of stimulus or elutant.** Representative dot plots present CD4 and CD8 marker expression before and after CD4 **(A)** and CD8 **(B)** cell selection.

### DETAILED DESCRIPTION

With the purpose of minimizing handling steps as well as selection-related contaminants, the inventor of the present application developed a new protocol to purify T cell populations of interest.

The inventor of the present application surprisingly found that target receptors on T cells, which are used for their purification, can be downregulated solely by incubating target cells at certain temperatures, in partuclar at about 24-37°C+/-5°C, thereby enabling their detachment from a selection agent. Surprisingly, the inventor of the present application found that downregulation of the target antigen can be effected without the need of additionaly stimulating the T cell. This finding stands in contrast to the teaching of WO 2021/084050, which teaches that downregulation of the target antigen and the resulting detachment from the selection agent is effected by activation induced detachment (AID), which means that activation of the cells are effected by contacting the T cells with an anti-CD3/anti-CD28 oligomeric stimulator reagent, which induces the downregulation of the target antigen, and results in detachment of T cells from a selection column by activation induced detachment. The inventor of the present application has however found, that contacting a T cell to an activation reagent is not required for inducing downregulation of the target antigen. As shown in **Examples 1-3** and **Figures 1-3****,** several T cell specific antigens, such as CD3, CD4, or CD8 can be downregulated solely by incubating the cells at about 24-37°C+/-5°C, resulting in their detachment from a CD3, CD4, or CD8-specific seletion reagents.

Compared to methods, which require the activation of T cells for their isolation, T cells obtained by means of the present invention have a much lower degree of activation. This is antvantageous, because in many instances activation is not required for downstream processing of isolated cells and may substantially limit experimentators.

A further advantage is that the method of the present invention can omit contamination of target cells certain reagents that interfere with downstream processes, such as oligomeric stimulation reagents. Hence, the methods of the present invention allows for reducing the number and/or intensity of washing and/or media exchange steps, which can be considered as cell stress factors and require considerable processing times.

Hence, the present invention is based on the concept that target cells from starting material (e.g. apheresis, buffy coat, whole blood etc.) are captured on selection agents (e.g., beads or bead-like particles loaded with antibodies or Fab fragments against target antigens present on the surface of target cell subset), thus separating target cells from the starting material. Subsequently, captured cells are released from selection agents upon temperature increase to 24-37°C+/-5°C in the presence or absence of cell culture media by inducing target antigen downregulation.

Released cells can be collected in an appropriate container downstream (e.g. cell culture bag, bioreactor) and processed accordingly (e.g. culture and expansion). This procedure enables seamless inclusion of an accelerated as well as large scale cell manufacturing processes. It is also strongly supporting integration and automation of cell manufacturing.

Hence, the present invention relates to a method of isolating one or more T cells, wherein the method comprises (a) contacting a plurality of T cells with a selection agent that is specific for a receptor on one or more of the T cells. In (a), the selection agent may be attached to a solid phase. Alternatively, in (a), the selection agent may be not attached to a solid phase when it is contacted to said plurality of T cells. In such cases, the method preferably further comprises a step of attaching the selection agent to a solid phase. Further, the method comprises (b) incubating the one or more T cells at about 19 °C to about 42°C for about 1 h to about 24 h, thereby inducing downregulation of said receptor on the T cell, resulting in relase of one or more of the plurality of the T cells from the selection agent. The steps (a) and (b) are conducted in absence of an oligomeric stimulatory reagent that comprises a specific binding partner for CD3 and/or a specific binding partner for CD28.

In cases where the selection agent is not attached to a solid phase when it is contacted to said plurality of T cells and the method further comprises a step of attaching the selection agent to a solid phase, the attaching can be conducted by any suitable means and methods known to the skilled person. For example, the attaching can be conducted by using an affinity tag system. For example, an affinity tag system can comprise a biotin, a biotin analog, or a streptavidin binding peptide on the one hand and a streptavidin, a streptavidin mutein, an avidin, or an avidin mutein on the other hand. For example, the selection agent can comprise a biotin, a biotin analog, or a streptavidin binding peptide and the attaching is conducted by contacting the selection agent with a solid phase comprising a streptavidin, a streptavidin mutein, an avidin, or an avidin mutein. As a further example, an affinity tag system can comprise an oligohistidine tag and divalent cations. For example, the selection agent can comprise an oligohistidine tag and the attaching is conducted by contacting the selection agent with a solid phase comprising a divalent cation.

The method of the present invention may further comprise (c) collecting one or more of the plurality of T cells from the solid phase, thereby generating a composition comprising one or more isolated T cells.

Such T cells isolated in step (c) are preferably unstimulated T cells. Stimulation status of T cells can be determined by assement of early activatin markers such as CD69 or Nur77.

A solid phase, to which the selection ragent is attached or can be attached, is typically any solid carrier that is suitable for isolation of T cells. In some cases, the solid phase may comprise a synthetic bead particle. Such synthetic bead particle can be magnetic or non-magnetic. Suitable beads for the isolation of T cells are well know in the art and are commercially available. Examples of synthetic bead particles include but are not limited to agarose beads or polystyrene beads. In cases where the solid phase comprises a synthetic bead particle, said solid phase is typically in solution, when the plurality of T cells is contacted with the seletion agent and/or when the selection agent is attached to the solid phase. The synthetic bead particle can then be isolated to enable the isolation of the one or more T cells attached that are attached to it.

In some cases, the solid phase may comprise a chromatography matrix. Generally, any chromatography matrix suitable for T cell isolation may be employed. Preferably, a suitable chromatography material is essentially innocuous, i.e. not detrimental to cell viability, such as when used in a packed chromatography column under desired conditions. Suitable chromatography matixes for the isolation of T cells are well know in the art and are commercially available. Examples of chromatography matixes include but are not limited to agarose or sepharose resins.

T cells or T lymphocytes, in particular the one or more T cells to be isolated, can be distinguished from other lymphocytes, such as B cells and natural killer cells (NK cells), by the presence of a T-cell receptor (TCR) on the cell surface. They are called T cells because they mature in the thymus (although some also mature in the tonsils). There are several subsets of T cells, each with a distinct function. In some cases, the one or more T cells are CD3+. In some cases, the one or more T cells are CD4+. In some cases, the one or more T cells are CD8+. In some cases, the one or more T cells are CD3+ and CD4+. In some cases, the one or more T cells are CD3+ and CD8+.

In the methods of the present invantion, steps (a) and (b) are preferably conducted in absence of a T cell stimulatory reagent. Said T cell stimulatory reagent is preferably characterized by being capable of delivering a stimulatory signal and/or proliferative in the T cell. Said stimulatory signal and/or proliferative signal is preferably delivered through a TCR/CD3 complex in a T cell, a CD3-containing complex in a T cell, and/or an ITAM-containing molecule in a T cell. Said T cell stimulatory reagent can be one that is capable of cross-linking a TCR/CD3 complex and one or more T cell co-stimulatory molecule(s). For example, a T cell stimulatory reagent preferably comprises one or more binding agent, that is specific for one or more targets selected from CD3, TCR, CD28, CD90 (Thy-1), CD95 (Apo-/Fas), CD137 (4-1BB), CD154 (CD40L), ICOS, CD27, OX40 or HYEM. Optionally, said T cell stimulatory reagent is oligomeric and comprises one or more binding agent specific for one or more targets selected from CD3, TCR, CD28, CD90 (Thy-1), CD95 (Apo-/Fas), CD137 (4-1BB), CD154 (CD40L), ICOS, CD27, OX40 or HYEM. Said T cell simulatory reagent can comprise binding agents that are specific for two or more, such as two, three, four, or even more, preferably two targets selected from CD3, TCR, CD28, CD90 (Thy-1), CD95 (Apo-/Fas), CD137 (4-1BB), CD154 (CD40L), ICOS, CD27, OX40 or HYEM. Said T cell stimulatory reagent may comprise binding agents specific for CD3 and/or CD28, which can be an oligomeric stimulatory reagent.

A stimulatory reagent can be or comprise one or more binding agents selected from the group consisting of antibody fragments, monovalent antibody fragments, proteinaceous binding molecules with immunoglobulin-like functions, molecules containing Ig domains, cytokines, ehemokines, aptamers, MHC molecules, MHO peptide complexes, receptor ligands, and binding fragments thereof. A stimulatory reagent can be or comprise an antibody fragment, which is preferably specific for any one of CD3, TCR, CD28, CD90 (Thy-1), CD95 (Apo-/Fas), CD137 (4-1BB), CD154 (CD40L), ICOS, CD27, OX40 or HYEM. A stimulatory agent can be or comprise a Fab fragment, which is preferably specific for for any one of CD3, TCR, CD28, CD90 (Thy-1), CD95 (Apo-/Fas), CD137 (4-1BB), CD154 (CD40L), ICOS, CD27, OX40 or HYEM. A stimulatory agent can be or comprise one selected from the group of divalent antibody fragments consisting of F(ab')₂ fragments and divalent single-chain Fv (scFv) fragments, which is preferably specific, such as monospecific or bispecific, for any one of CD3, TCR, CD28, CD90 (Thy-1), CD95 (Apo-/Fas), CD137 (4-1BB), CD154 (CD40L), ICOS, CD27, OX40 or HYEM. A stimulatory agent can be or comprise a monovalent antibody fragment selected from the group consisting of Fab fragments, Fv fragments, and scFvs, which is preferably specific for for any one of CD3, TCR, CD28, CD90 (Thy-1), CD95 (Apo-/Fas), CD137 (4-1BB), CD154 (CD40L), ICOS, CD27, OX40 or HYEM. A stimulatory agent can be or comprise a proteinaceous binding molecule with antibody-like binding properties, selected from the group consisting of aptamers, muteins based on a polypeptide of the lipocalin family, glubodies, proteins based on the ankyrin scaffold, proteins based on the crystalline scaffold, adnectins, and avimers, which is preferably specific for for any one of CD3, TCR, CD28, CD90 (Thy-1), CD95 (Apo-/Fas), CD137 (4-1BB), CD154 (CD40L), ICOS, CD27, OX40 or HYEM.

A stimulatory reagent can further comprise biotin, a biotin analog that reversibly binds to a streptavidin or avidin, a streptavidin binding peptide selected from the group consisting of WSHPQFEK (SEQ ID NO: 1), SAWSHPQFEKGGGSGGGSGGGSWSHPQFEK (SEQ ID NO: 2), WSHPQFEKGGGSGGGSGGGSWSHPQFEK (SEQ ID NO: 3), SAWSHPQFEKGGGSGGGSGGSAWSHPQFEK (SEQ ID NO: 4), WSHPQFEKGGGSGGGSWSHPQFEK (SEQ ID NO: 5) and WSHPQFEKGGGSGGGSGGSAWSHPQFEK (SEQ ID NO: 6), a calmodulin binding peptide that reversibly binds to calmodulin, a FLAG peptide that reversibly binds to an antibody binding the FLAG peptide, and an oligohistidine tag that reversibly binds to an antibody binding the oligohistidine tag.

A stimulatory reagent may be or comprise an oligomeric stimulatory reagent comprising (i) a plurality of streptavidin or streptavidin mutein molecules and (ii) one or more stimulatory agent capable of delivering a stimulatory signal in one or more T cells, where the size of the oligomeric stimulatory reagent comprises i) a radius of greater than 50 nm, ii) a molecular weight of at least 5 x 10⁶ g/mol; and/or (iii) at least 100 streptavidin or streptavidin mutein tetramers per oligomeric stimulatory reagent. In some cases, the streptavidin mutein comprises the amino acid sequence Val⁴⁴-Thr⁴⁵-Ala⁴⁶-Arg⁴⁷ (SEQ ID NO: 7) or lle⁴⁴-Gly⁴⁵-Ala⁴⁶-Arg⁴⁷ (SEQ ID NO: 8) at sequence positions corresponding to positions 44 to 47 with reference to positions in streptavidin in the sequence of amino adds set forth in SEQ ID NO: 9.

A stimulatory reagent may be or comprise an oligomeric stimulatory reagent comprising a plurality of binding agents specific for one or more, such as two, three, four, or even more, preferably two targets selected from CD3, TCR, CD28, CD90 (Thy-1), CD95 (Apo-/Fas), CD137 (4-1BB), CD154 (CD40L), ICOS, CD27, OX40 or HYEM, which are reversibly or irreversibly (e.g. covalently) bound to a particle having i) a radius of greater than 50 nm, ii) a molecular weight of at least 5 x 10⁶ g/mol. Such stimulatory reagent may comprise a plurality of at least 10, at least 20, at least 30, at least 40, at least 50, at least 60, at least, 70, at least 80, at least 90, or at least 100 of the aforementioned binding agents.

A stimulatory reagent can be capable of delivering a stimulatory signal and/or proliferative signal, wherein the stimulatory signal and/or proliferative signal is through a TCR/CD3 complex in a T cell, a CD3-containing complex in a T cell, and/or an ITAM containing molecule in a T cell. The stimulatory reagent can comprise a first binding agent capable of delivering a first stimulatory signal and a second binding agent capable of enhancing, dampening, or modifying the first stimulatory signal of the first stimulatory agent. In some cases, the second biding agent can be capable of specifically binding to a costimulatory molecule on the one or more T cells. In some cases, the costimulatory molecule can be selected from among CD28, CD90 (Thy-1), CD95 (Apo-/Fas), CD137 (4-1BB), CD154 (CD40L), ICOS, LAT, CD27, OX40 or HVEM. In preferred cases, the second stimulatory agent can be capable of specifically binding to CD28 and/or the costimulatory molecule is CD28. In some cases, the first binding agent can be capable of specifically binding to CD3 or TCR. In preferred cases, the first binding agent is capable of specifically binding to CD3.

In preferred cases, the stimulator reagent comprises one or more first binding agents that can specifically bind CD3 and one or more second binding agents can specifically bind CD28. In some cases, the first binding agent can comprise an antibody that binds to a first target, such as CD3 or TCR, preferably CD3, and the second binding agent can comprise an antibody that binds to a second target, such as any one of CD28, CD90 (Thy-1), CD95 (Apo-/Fas), CD137 (4-1BB), CD154 (CD40L), ICOS, LAT, CD27, OX40 or HVEM, preferably CD28. In some cases, the T cell stimulatory reagent is antibody based. In some cases, the first binding agent can comprise a monovalent antibody fragment that binds to a first target, such as CD3 or TCR, preferably CD3, and the second binding agent can comprise a monovalent antibody fragment that binds to a second target, such as any one of CD28, CD90 (Thy-1), CD95 (Apo-/Fas), CD137 (4-1BB), CD154 (CD40L), ICOS, LAT, CD27, OX40 or HVEM, preferably CD28. The monovalent antibody fragment can be selected from the group consisting of a Fab fragment, an Fv fragment, and a single-chain Fv fragment (scFv). In some cases, the monovalent antibody fragment is a Fab fragment. In some cases, the T cell stimulatory agent is Fab-based. Preferably, the first binding agent can be an anti-CD3 Fab and the second binding agent can be an anti-CD28 Fab.

In the methods of the present invention, detaching the cells from the selection agent is preferably conducted without detaching the selection agent from the solid phase. In particular, it is preferred that at least steps (a) and (b) are conducted without disrupting the attachment of the specific binding agent to the solid phase. It is further preferred that step (c) is conducted without disrupting the attachment of the specific binding agent to the solid phase.

In some cases, the the solid phase to which the selection agent is bound provides a reversible system in which the selection agent is reversibly associated with the solid phase. Exemplary reversible systems for selection of cells by synthetic bead particles or chromatography are know to the skilled person and include those described in WO 2013/124474. In some cases, the reversible system can employ a reagent composed of streptavidin mutein molecules that reversibly bind to the selection agent via a streptavidin-binding peptide binding partner contained by the selection agent. In some cases, the reversible system can employ a divalent cation that reversibly binds to an oligohistidine tag. In such cases, adding an elution reagent, which can a free binding partner or competition agent (also called competition substance) can disrupt the binding between the selection agent and the solid phase, thereby reversing binding of the selection agent from the reagent and releasing the immobilized cells free from the selection reagent. For instance, in the case of a streptavidin mutein/streptavidin binding peptide system, an exemplary elution reagent is biotin (e.g., D-biotin) or a biotin analog. In cases where divalent cations and oligohistidine tags form the reversible system, the elution reagent can be imidazole. Further reversible systems as well as corresponding elution reagents are known to the skilled person. In any case, it is preferred that at least steps (a) and (b) are conducted without addition of an elution reagent, wherein the elution reagent is preferably selected from the group consisting of: biotin, D-biotin, or imidazole. It is further preferred that step (c) is conducted without addition of an elution reagent, wherein the elution reagent is preferably selected from the group consisting of: biotin, D-biotin, or imidazole. It is also preferred that in the methods of the present disclosure, steps (a) and/or (b) are conducted without disrupting the attachment of the specific binding agent to the solid phase.

According to the present disclosure, the incubation in step (b) can be carried out at any temperature that is sutibable for inducing downregulation of said receptor on the T cell. For example, the temperature may be from about 19° to about 42°C. In some cases, the incubation in step (b) is carried out at about 24°C to about 40°C, such as about 30°C to about 39°C, preferably about 35°C to about 38°C, preferably about 37°C.

According to the present disclosure, the incubation in step (b) can be carried out for any period of time that is sutibable for inducing downregulation of said receptor on the T cell. For example, the period of time can be from about 1 h to about 24 h. In some cases, the incubation in step (b) can be carried out for about 1 h to about 18 h, such as about 1 h to about 12 h, about 2 h to about 8 h, about 2 h to about 6 h, or preferably about 2 h to about 4 h.

In some cases, the provided methods include isolating cells or populations thereof from biological samples, such as those obtained from or derived from a subject, such as one having a particular disease or condition or in need of a cell therapy or to which cell therapy will be administered. In some cases, the subject is a human, such as a subject who is a patient in need of a particular therapeutic intervention, such as the adoptive cell therapy for which cells are being isolated, processed, and/or engineered. Accordingly, in some cases, the plurality of T cells to be contacted with a a selection agent ins step (a) are primary cells, e.g., primary human cells. The plurality of T cells to be contacted with a selection agent in step (a) may be from and/or be comprised in a sample, preferably a biological sample. Said sample may be or comprise tissue, fluid, and other samples taken directly from the subject. The biological sample can be a sample obtained directly from a biological source or a sample that is processed.

In some cases, the sample may be or comprise blood or a blood-derived sample, or an apheresis or leukapheresis product. The samples, in some cases, contain lymphocytes, including T cells, monocytes, granulocytes, B cells, other nucleated white blood cells, red blood cells, and/or platelets, and in some aspects contains cells other than red blood cells and platelets. In some cases, the sample contains cells from the circulating blood of a subject, which can, e.g., be obtained by apheresis or leukapheresis. The samples may contain lymphocytes, including T cells, monocytes, granulocytes, B cells, other nucleated white blood cells, red blood cells, and/or platelets, and in some aspects contains cells other than red blood cells and platelets.

In some cases, the sample is a sample containing T cells. In some cases, the sample is or comprise a whole blood sample, a buffy coat sample, a peripheral blood mononuclear cell (PBMC) sample, an unfractionated T cell sample, a lymphocyte sample, a white blood cell sample, an apheresis product, or a leukapheresis product. In some cases, the sample is an apheresis product. In some cases, the sample is a leukapheresis product.

In some cases, the blood cells collected from the subject are washed, e.g., to remove the plasma fraction and to place the cells in an appropriate buffer or media for subsequent processing steps. In some cases, the cells are washed with phosphate buffered saline (PBS), In some cases, the wash solution lacks calcium and/or magnesium and/or many or all divalent cations. After washing, cells can be resuspended in a variety of biocompatible buffers, such as, for example, Ca²⁺/Mg²⁺ free PBS. In certain cases, components of a blood cell sample are removed and the cells directly resuspended in culture media. In some cases, the sample containing cells (e.g., an apheresis product or a leukapheresis product) is washed in order to remove one or more anti-coagulants, such as heparin, added during apheresis or leukapheresis.

In some cases, the sample, e.g., a whole blood sample, a buffy coat sample, a peripheral blood mononuclear cells (PBMC) sample, an unfractionated T cell sample, a lymphocyte sample, a white blood cell sample, an apheresis product, or a leukapheresis product, is cryopreserved and/or eryoprotected (e.g., frozen) and then thawed prior to any steps for isolating one or more T cells. In particular cases, an apheresis product or a leukapheresis product is cryopreserved and/or eryoprotected (e.g., frozen) and then thawed before being subject to a cell isolation step (e.g., a T cell isolation step) as described herein.

The thawed sample can be subjected to dilution (e.g., with a serum-free medium) and/or wash (e.g., with a serum-free medium), which in some cases can remove or reduce unwanted or undesired components. In some cases, the dilution and/or wash removes or reduces the presence of a cryoprotectant, e.g. DMSO, contained in the thawed sample, which otherwise may negatively impact cellular viability, yield, recovery upon extended room temperature exposure. In some embodiments, the dilution and/or wash allows media exchange of a thawed cryopreserved product into a serum-free medium, such as one described herein or in WO 2019/113556, which is incorporated herein by reference.

In some cases, the receptor on the one or more of the T cells is or comprises a T cell coreceptor. In some cases, the receptor on the one or more of the T cells is or comprise a member of a T cell antigen receptor complex. In some cases, the receptor on the one or more of the T cells is or comprise a TCR alpha chain and/or beta chain. In some cases, the receptor on the one or more of the T cells is or comprises CD3. In some cases, the receptor on the one or more of the T cells is or comprises a CD3 chain, such as a CD3 gamma chain, a CD3 delta chain, a CD3 epsilon chain, and/ or a CD3 zeta chain. In some cases, the receptor on the one or more of the T cells is or comprises a CD3 gamma chain. In some cases, the receptor on the one or more of the T cells is or comprises a CD3 delta chain. In some cases, the receptor on the one or more of the T cells is or comprises a CD3 epsilon chain. In some cases, the receptor on the one or more of the T cells is or comprises a CD3 zeta chain. In some cases, the receptor on the one or more of the T cells is or comprises a CD8. In some cases, the receptor on the one or more of the T cells is or comprises a CD4. In some cases, the receptor on the one or more of the T cells is or comprises a CD45RA. In some cases, the receptor on the one or more of the T cells is or comprises a CD27. In some cases, the receptor on the one or more of the T cells is or comprises a CD28. In some cases, the receptor on the one or more of the T cells is or comprises a CD137 (4-1BB). In some cases, the receptor on the one or more of the T cells is or comprises a CCR7. In some cases, the receptor on the one or more of the T cells is or comprises a CD62L. In some cases, the receptor on the one or more of the T cells is or comprises a CD25. In preferred cases, the receptor on the one or more of the T cells is selected from the group consisting of CD3, CD4, and CD8. Preferably, the the receptor on the one or more of the T cells is CD3.

In some cases, the specific binding between the selection agent and the receptor on one or more of the T cells does not induce a signal to said one or more of th T cells. Such signal may include a stimulatory signal, an activating signal, and/or a proliferative signal. Preferably, the specific binding between the selection agent and the receptor on one or more of the T cells does not induce a proliferative signal to said one or more of th T cells.

According to the present disclosure, the selection agent may comprise at least an antigen-binding fragment of an antibody, which specifically binds to receptor on the T cell.

The term "antibody" generally refers to a proteinaceous binding molecule with immunoglobulin-like functions. Typical examples of an antibody are, but are not limited to, immunoglobulins, as well as derivatives or functional fragments thereof which still retain the binding specificity. Techniques for the production of antibodies are well known in the art. The term "antibody" also includes immunoglobulins (Ig's) of different classes (i.e. IgA, IgG, IgM, IgD, IgE, IgY etc.) and subclasses (such as IgG1, lgG2 etc.), even if recombinantly produced in foreign hosts using techniques known to those skilled in the arts. Illustrative examples of an antibody are full length immunoglobulins, Fab fragments, F(ab')₂, Fv fragments, single-chain Fv fragments (scFv), diabodies or domain antibodies (Holt LJ et al., Trends Biotechnol. 21(11), 2003, 484-490). Domain antibodies may be single domain antibodies, single variable domain antibodies or immunoglobulin single variable domain having only one variable domain, which may be VH or VL, that specifically bind an antigen or epitope independently of other V regions or domains. A preferred single domain antibody is a VHH domain of a heavy chain only antibody. Such an immunoglobulin single variable domain may not only encompass an isolated antibody single variable domain polypeptide, but also a larger polypeptide that includes or consists of one or more monomers of an antibody single variable domain polypeptide sequence.

In some cases, the selection agent comprises a mono-, bi-, or multivalent antibody, antibody construct, or antibody fragment that binds to the receptor on the T cell. The receptor on the T cell may preferably be CD3, CD8, or CD4. The selection agent may comprises a immunoglobulin, such as an IgA, IgG, IgM, IgD, IgE, or IgY.

As used herein, the term "antibody construct" refers to a molecule in which the structure and/or function is/are based on the structure and/or function of an antibody, e.g., of a full-length or whole immunoglobulin molecule and/or is/are drawn from the variable heavy chain (VH) and/or variable light chain (VL) domains of an antibody or fragment thereof. An antibody construct is hence capable of binding to its specific target or antigen. Furthermore, the binding region of an antibody construct comprises the minimum structural requirements of an antibody which allow for the target binding. A binding domain of an antibody construct preferably comprises a designated group of CDRs. Those CDRs can be comprised in the framework of an antibody light chain variable region (VL) and an antibody heavy chain variable region (VH).

The term "variable" refers to the portions of the immunoglobulin domains that exhibit variability in their sequence and that are involved in determining the specificity and binding affinity of a particular antibody (i.e., the "variable domain(s)"). Variability is not evenly distributed throughout the variable domains of antibodies; it is concentrated in sub-domains of each of the heavy and light chain variable regions. These sub-domains are called "hypervariable regions", "HVR," or "HV," or "complementarity determining regions" (CDRs). The more conserved (i.e., non-hypervariable) portions of the variable domains are called the "framework" regions (FR). The variable domains of naturally occurring heavy and light chains each include four FR regions, largely adopting a β-sheet configuration, connected by three hypervariable regions, which form loops connecting, and in some cases forming part of, the β-sheet structure. The hypervariable regions in each chain are held together in close proximity by the FR and, with the hypervariable regions from the other chain, contribute to the formation of the antigen-binding site. Generally, naturally occurring immunoglobulins include six CDRs (see below); three in the VH (H1, H2, H3), and three in the VL (L1, L2, L3). In naturally occurring immunoglobulins, H3 and L3 display the most diversity of the six CDRs, and H3 in particular is believed to play a unique role in conferring fine specificity to immunoglobulins. Immunoglobulins naturally devoid of light chains, however, include three CDRs that are in the VHH region. The constant domains are not directly involved in antigen binding, but exhibit various effector functions, such as, for example, antibody-dependent, cell-mediated cytotoxicity and complement activation.

The valence of an antibody is generally an expression of the number of antigen-binding sites for one molecule of any given antibody or the number of antibody-binding sites for any given antigen. Most antibody molecules, and those belonging to the IgG, IgA, IgD and IgE immunoglobulin classes, have two antigen-binding sites per molecule. In general, a monovalent antibody comprises a single antigen binding site. Examples for a monovalent antibody are a single domain antibody, a VHH domain, a single Fab fragment, a Fv fragment, a scFv fragment, or a single VH or VL domain.

An antigen-binding fragment of an antibody, generally comprises a VH and/or a VL region derived from said antibody, or, in case the antigen-binding fragment is from an antibody devoid of light chains, an antigen-binding fragment comprises a variable region, such as a VHH region. In some cases, an antigen-binding fragment may be or comprise a Fab fragment. In some cases, an antigen-binding fragment may be or comprise a scFv.

The terms "Fab", "Fab region", "Fab portion" or "Fab fragment" are understood to define a polypeptide that includes a VH, a CH1, a VL, and a CL immunoglobulin domain. Fab may refer to this region in isolation, or this region in the context of an antibody molecule according to the invention, as well as a full-length immunoglobulin or immunoglobulin fragment. Typically, a Fab region contains an entire light chain of an antibody. A Fab region can be taken to define "an arm" of an immunoglobulin molecule. It contains the epitope-binding portion of that Ig. The Fab region of a naturally occurring immunoglobulin can be obtained as a proteolytic fragment by a partial papain-digestion. A "F(ab')₂ portion" is the proteolytic fragment of a partially pepsin-digested immunoglobulin. A "Fab' portion" is the product resulting from reducing the disulfide bonds of an F(ab')₂ portion. As used herein the terms "Fab", "Fab region", "Fab portion" or "Fab fragment" may further include a hinge region that defines the C-terminal end of the antibody arm (cf. above). This hinge region corresponds to the hinge region found C-terminally of the CH1 domain within a full length immunoglobulin at which the arms of the antibody molecule can be taken to define a Y. The term hinge region is used in the art because an immunoglobulin has some flexibility at this region.

An "Fv" or "Fv fragment" consists of only the V_{L} and V_{H} domains of a "single arm" of an immunoglobulin. Thus an "Fv" is the minimum antibody fragment which contains a complete antigen-recognition and binding site. A "two-chain" Fv fragment consists of a dimer of one heavy- and one light-chain variable domain in tight, non-covalent association. A single-chain Fv species (scFv) includes a V_{H} and a V_{L} domain of an immunoglobulin, with these domains being present in a single polypeptide chain in which they are covalently linked to each other by a flexible peptide linker. Typically, in a scFv fragment the variable domains of the light and heavy chain associate in a dimeric structure analogous to that in a two-chain Fv species. In single chain Fv fragments, it is possible to either have the variable domain of the light chain arranged at the N-terminus of the single polypeptide chain, followed by the linker and the variable domain of the heavy chain arranged at the C-terminus of the polypeptide chain or vice versa, having the variable domain of the heavy chain arranged on the N-terminus and the variable domain of the light chain at the C-terminus with the peptide linker arranged in between. The peptide linker can be any flexible linker known in the art, for example, made from glycine and serine residues. It is also possible to additionally stabilize the domain association between the V_{H} and the V_{L} domain by introducing disulfide bonds into conserved framework regions (see Reiter et al. Stabilization of the Fv fragments in recombinant immunotoxins by disulfide bonds engineered into conserved framework regions, Biochemistry 1994, 33, 6551-5459). Such scFv fragments are also known as disulfide-stabilized scFv fragments (ds-scFv).

An antibody according to the invention may be produced using any known and well-established expression system and recombinant cell culturing technology, for example, by expression in bacterial hosts (prokaryotic systems), or eukaryotic systems such as yeasts, fungi, insect cells or mammalian cells. An antibody molecule of the present invention may be produced in transgenic organisms such as a goat or a plant. An antibody may also be produced by chemical synthesis.

An antibody or antibody molecule/fragment is said to "specifically" bind to an antigen when it recognizes its target antigen within a complex mixture of proteins and/or macromolecules. Typically, the antibody is capable of specifically interacting with and/or binding to its target but does not essentially bind to another epitope or antigen. Antibodies are said to "bind to the same epitope" if the antibodies cross-compete so that only one antibody can bind to the epitope at a given point of time, i.e. one antibody prevents the binding or modulating effect of the other.

In some cases, the receptor on the one or more of the T cells may be CD4 and the selection agent specifically binds CD4. The selection agent that specifically binds CD4 may be selected from the group consisting of an anti-CD4 antibody, a divalent antibody fragment of an anti-CD4 antibody, a monovalent antibody fragment of an anti-CD4-antibody, and a proteinaceous CD4 binding molecule with antibody-like binding properties. In some cases, an anti-CD4-antibody, such as a divalent antibody fragment or a monovalent antibody fragment (eg. CD4 Fab fragment) can be derived from antibody 13B8.2 or a functionally active mutant of 13B8.2 that retains specific binding for CD4. For example, exemplary mutants of antibody 13B8.2 or m13B8.2 are described in U.S. Patent Nos. 7,482,000, U.S. Patent Appl. No. US2014/0295458 or International Patent Application No. WO2013/124474; and Bes, C, et al J Biol Chem 278, 14265-14273 (2003). The mutant Fab fragment termed "ml3B8.2" carries the variable domain of the CD4 binding murine antibody 13B8.2 and a constant domain containing constant human CH1 domain of type gamma for the heavy chain and the constant human light chain domain of type kappa, as described in US Patent 7,482,000. In some cases, the anti-CD4 antibody, e.g. a mutant of antibody 13B8.2, contains the amino acid replacement H91A in the variable light chain, the amino acid replacement Y92A in the variable light chain, the amino acid replacement H35A in the variable heavy chain and/or the amino acid replacement R53A in the variable heavy chain, each by Kabat numbering. In some cases, compared to variable domains of the 13B8.2 Fab fragment in m13B8.2 the His residue at position 91 of the light chain is mutated to Ala and the Arg residue at position 53 of the heavy chain is mutated to Ala. In some cases, the selection agent comprises an anti-CD4 Fab fragment. In some cases, the anti-CD4 Fab fragment comprises a variable heavy chain having the sequence set forth by SEQ ID NO: 10 and a variable light chain having the sequence set forth by SEQ ID NO: 11. In some embodiments, the anti-CD4 Fab fragment comprises the CDRs of the variable heavy chain having the sequence set forth by SEQ ID NO: 10 and the CDRs of the variable light chain having the sequence set forth by SEQ ID NO: 11.

In some cases, the receptor on the one or more of the T cells may be CD8 and the selection agent specifically binds CD8. In some aspects, the selection agent that specifically binds CD8 may be selected from the group consisting of an anti-CD8-antibody, a divalent antibody fragment of an anti-CD8 antibody, a monovalent antibody fragment of an anti-CD8-antibody, and a proteinaceous CDS binding molecule with antibody-like binding properties. In some cases, an anti-CD8-antibody, such as a divalent antibody fragment or a monovalent antibody fragment (e.g. CD8 Fab fragment) can be derived from antibody OKT8 (eg. ATCC CRL-8014) or a functionally active mutant thereof that retains specific binding for CD8. In some cases, the selection agent comprises an anti-CD8 Fab fragment. In some cases, the anti-CD8 Fab fragment comprises a variable heavy chain having the sequence set forth by SEQ ID NO: 12 and a variable light chain having the sequence set forth by SEQ ID NO: 13. In some cases, the anti-CD8 Fab fragment comprises the CDRs of the variable heavy chain having the sequence set forth by SEQ ID NO: 12 and the CDRs of the variable light chain having the sequence set forth by SEQ ID NO: 13.

In some cases, the receptor on the one or more of the T cells may be CD3 and the selection agent specifically binds CD3. In some cases, the selection agent that specifically binds CD3 may be selected from the group consisting of an anti-CD3-antibody, a divalent antibody fragment of an anti-CD3 antibody, a monovalent antibody fragment of an anti-CD3-antibody, and a proteinaceous CD3 binding molecule with antibody-like binding properties. In some embodiments, an anti-CD3 antibody, such as a divalent antibody fragment or a monovalent antibody fragment (e.g. CD3 Fab fragment) can be derived from antibody OKT3 (eg. ATCC CRL-8001; see eg., Stemberger et at. PLoS One. 2012; 7(4): e35798) or a functionally active mutant thereof that retains specific binding for CD3. In some cases, the selection agent comprises an anti-CD3 Fab fragment. In some cases, the anti-CD3 Fab fragment comprises a variable heavy chain having the sequence set forth by SEQ ID NO: 14 and a variable light chain having the sequence set forth fay SEQ ID NO: 15. In some embodiments, the anti-CD3 Fab fragment comprises the CDRs of the variable heavy chain having the sequence set forth by SEQ ID NO: 14 and the CDRs of the variable light chain having the sequence set forth by SEQ ID NO: 15.

The selection agent may be directly or indirectly bound to the solid phase. In some cases, the selection agent is bound indirectly to the solid phase through a selection reagent to which the selection agent reversibly or irreversibly binds. In some cases, the the selection reagent is or comprises streptavidin, avidin, a mutein of streptavidin that irreversibly or reversibly binds biotin, a biotin analog or a biologically active fragment thereof; a mutein of avidin or streptavidin that irreversibly or reversibly binds a streptavidin-binding peptide; a reagent that contains at least two chelating groups K, wherein the at least two chelating groups are capable of binding to a transition metal ion; an agent capable of binding to an oligohistidine affinity tag; an agent capable of binding to a glutathione-S-transferase; calmodulin or an analog thereof; an agent capable of binding to calmodulin binding peptide (CBP); an agent capable of binding to a FLAG-peptide; an agent capable of binding to an HA-tag; an agent capable of binding to maltose binding protein (MBP); an agent capable of binding to an HSV epitope; an agent capable of binding to a myc epitope; or an agent capable of binding to a biotinylated carrier protein.

In some cases, the selection reagent is or comprises a streptavidin mutein or an avidin mutein that reversibly binds to biotin or a biologically active fragment. In some cases, the selection reagent is or contains a streptavidin mutein or an avidin mutein that reversibly binds to a streptavidin-binding peptide. In some embodiments, the streptavidin or streptavidin mutein molecules reversibly bind to or are capable of reversibly binding to biotin, a biotin analog or a streptavidin-binding peptide.

In some cases, the selection reagent is or comprises a streptavidin or an avidin that essentially irreversibibly binds to biotin or a biologically active fragment.

In some cases, the selection agent is covalently bound to the solid phase.

According to the present disclosure, the methods disclosed herein may further comprise: after the initiation of the incubation, such as after the incubation, collecting one or more of the plurality of T cells from the solid phase. By this, a composition comprising one or more isolated T cells may be generated. The one or more of the plurality of T cells may be collected from the stationary phase within 24 hours of the initiation of the incubation. The one or more of the plurality of T cells can be collected from the solid phase within 4 hours, within 4.5 hours, within 5 hours, within 6 hours, within 7 yours, within 8 hours, within 9 hours, within 10 hours, within 11 hours, or within 12 hours after the initiation of the incubation. In preferred cases, the one or more of the plurality of T cells can be collected from the solid phase about 1 h to about 18 h, preferably about 1 h to about 12 h, preferably about 2 h to about 8 h, preferably about 2 h to about 6 h, preferably about 2 h to about 4 h after the initiation of the incubation. The collection of one or more of the plurality of T cells can be by any suitable method known to the skilled person. For example, collecting the one or more of the plurality of T cells from the the solid phase may be by filtration to separate the one or more of the plurality of T cells from the solid phase, e.g. in cases where the solid phase comprises synthetic beads. Collecting the one or more of the plurality of T cells from the the solid phase can also be by gravity flow. Said collecting by gravity flow can comprise adding media, such as a wash media, to the solid phase (e.g. a chromatography column). In any of the preceding cases, the collecting step can be performed without the addition of a competition agent or free binding agent to elute the one or more of the plurality of T cells from the solid phase. In any of the preceding cases, the (wash media) may not comprise a competition agent or free binding agent to elute the one or more of the plurality of T cells from the solid phase.

As mentioned before, the collecting step may generate a composition comprising one or more isolated T cells. In some cases, said composition does not comprise a competition agent or free binding agent.

In some cases, the competition agent or free binding agent can be an agent that competes for binding with a streptavidin binding peptide of the selection agent to a streptavidin mutein immobilized on the stationary phase. In some cases, the competition agent or free binding agent can be a biotin or a biotin analog, optionally wherein the biotin analog is D-biotin. In some embodiments, the competition agent or free binding agent can be D-biotin.

The methods of isolating one or more T cells disclosed herein, may further comprise the step of introducing a recombinant nucleic acid molecule into the isolated one or more T cells, including CD3+ T cells, CD4+ T cells, and/or CD8+ T cells, thereby producing one or more engineered T cells. The recombinant nucleic acid molecule preferably encodes a recombinant protein. In certain cases, the methods provided herein are used in connection with manufacturing, generating, or producing a cell therapy. In some cases, the method disclosed herein results in production of one or more engineered T cells, and include one or more of steps for isolating one or more T cells from a sample obtained from a subject, optionally incubating the cells under stimulatory conditions, and genetically engineering the cells. The methods disclosed herein may include processing steps carried out in an order in one or more T cells, e.g. primary CD4+ and/or CD8+ T cells, are isolated as disclosed herein, such as selected or separated, from a biological sample, and subsequently genetically engineered to introduce a recombinant polynucleotide encoding a recombinant protein, such as a recombinant receptor, into the cells, such as by transduction or transfection, thereby obtaining one or more engineered T cells. The one or more engineered T cells can then be collected, harvested, or filled into a container, e.g., a bag or vial, as an output population. The output population preferably comprises one or more engineered T cells. In some cases, the one or more engineered T cells can be re-introduced into the same subject, optionally after cryopreserving and storing the cells. In some cases, the one or more engineered T cells are suitable for use in a therapy, e.g., an autologous cell therapy.

As disclosed herein, one or more recombinant nucleic acid molecule can be introduced into the isolated one or more T cells, which can encode a recombinant protein, e.g., a (recombinant) receptor. Among the receptors are antigen receptors and receptors containing one or more component thereof. The recombinant receptor may include chimeric receptors, such as those containing ligand-binding domains or binding fragments thereof and intracellular signaling domains or regions, functional non-TCR antigen receptors, chimeric antigen receptors (CARs), T cell receptors (TCRs), such as recombinant or transgenic TCRs, chimeric autoantibody receptor (CAAR) and components of any of the foregoing. The recombinant receptor, such as a CAR, generally includes the extracellular antigen (or ligand) binding domain linked to one or more intracellular signaling components, in some aspects via linkers and/or transmembrane domain(s). In some examples, said one or more recombinant nucleic acid molecules can encode two or more receptors that contain different components, domains or regions. In some examples, two or more receptors allows spatial or temporal regulation or control of specificity, activity, antigen (or ligand) binding, function and/or expression of the recombinant receptors.

In some examples, chimeric antigen receptors contain one or more domains that combine a ligand-binding domain (e.g. antibody or antibody fragment) that provides specificity for a desired antigen (e.g., tumor antigen) with intracellular signaling domains. The intracellular signaling domain is preferably an activating intracellular domain portion, such as a T cell activating domain, providing a primary activation signal. In some examples, the intracellular signaling domain contains or additionally contains a costimulatory signaling domain to facilitate effector functions. Chimeric receptors when genetically engineered into immune cells can preferably modulate T cell activity, and, in some cases, can modulate T cell differentiation or homeostasis, thereby resulting in genetically engineered cells with improved longevity, survival and/or persistence in vivo, such as for use in adoptive cell therapy methods.

The chimeric receptors, such as CARs, may generally include an extracellular antigen binding domain, such as a portion of an antibody molecule, generally a variable heavy (VH) chain region and/or variable light (VL) chain region of the antibody, e.g., an scFv antibody fragment.

The antigen receptor may comprise an intracellular domain linked directly or indirectly to the extracellular domain. In some examples, the chimeric antigen receptor includes a transmembrane domain linking the extracellular domain and the intracellular signaling domain. For example, the intracellular signaling domain may comprise an ITAM. For example, the antigen recognition domain (e.g. extracellular domain) generally can be linked to one or more intracellular signaling components, such as signaling components that mimic activation through an antigen receptor complex, such as a TCR complex, in the case of a CAR, and/or signal via another cell surface receptor. In some examples, the CAR comprises a transmembrane domain linked or fused between the extracellular domain (e.g. a scFv) and intracellular signaling domain. Thus, in some examples, the antigen-binding component (e.g., antibody or fragment or variant thereof) is linked to one or more transmembrane and intracellular signaling domains.

The antigen receptor, e.g. CAR, may generally include at least one intracellular signaling component or components. In some examples, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta and CD3 epsilon. In some examples, cytoplasmic signaling molecule(s) in the CAR contain(s) a cytoplasmic signaling domain, portion thereof, or sequence derived from CD3 zeta. In some examples, the receptor includes an intracellular component of a TCR complex, such as a TCR/CD3 chain that mediates T-cell activation and cytotoxicity, e.g., CD3 zeta chain.

The chimeric antigen receptor may also contain an intracellular domain of a T cell costimulatory molecule. For example, the CAR may include a signaling domain and/or transmembrane portion of a costimulatory receptor, such as CD28, 4-1BB, OX40, DAP10, and ICOS. In some examples, the same CAR includes both the activating and costimulatory components. The chimeric antigen receptor may contain an intracellular domain derived from a T cell costimulatory molecule or a functional variant thereof, such as between the transmembrane domain and intracellular signaling domain. The T cell costimulatory molecule may be selected from the group consistign of CD28 and 4-1BB, preferably CD28.

The transmembrane domain can generally be derived either from a natural or from a synthetic source. Where the source is natural, the domain can be derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. For example, the transmembrane domain may comprise a transmembrane portion of CD28.

In some cases, the antigen targeted by the antigen receptor, such as CAR or TCR, is a polypeptide. In some cases, it is a carbohydrate or other molecule. In some cases, the antigen is selectively expressed or overexpressed on cells of the disease or condition, e.g., the tumor or pathogenic cells, as compared to normal or non-targeted cells or tissues. In other cases, the antigen is expressed on normal cells and/or is expressed on the engineered cells.

The antigen receptor discloses herein can be specific for a target, which is an antigen that is a polypeptide, a carbohydrate or other molecule. The antigen is preferably selectively expressed or overexpressed on cells of the disease or condition, e.g., the tumor or pathogenic cells, as compared to normal or non-targeted cells or tissues. The target can be overexpressed on the surface of the target cell, such as tumor or cancer cell, or overexpressed and secreted by the target cell, such as tumor cell. Said target antigen of the antigen receptor according to the present disclosure may comprise, but is not limited to a target selected from the group consisting of CD19, CD20, EpCAM, GPRC5D, DLL3, gp100, MCSP, CD22, CD30, CD33, CD52, CD70, CD74, CD79b, CD123, CLL1, BCMA, FCRH5, EGFR, EGFRvlll, HER2/neu, CD66e, EphA2, GD2, Trp1, B7H3 (CD276), PSMA, CEA, GPA33, GPC3, PD-L1, α-Folate receptor, CAIX, CD4, CD5, CD7, CD10, CD23, CD24, CD37, CD38, CD44v7/8, EGP-2, EGP-40, Erb-B2/3/4, FBP, Flt3, L1CAM, ICAM-1, TM4SF, AXL, CS1, ALPP, c-MET, MG7, CD131, CD138, FAP, CD171, Fetal acethylcholine receptor, GD3, HMW-MAA, IL-11Rα, IL-13Rα2, KDR, k-light chain, Lewis Y, L1-cell adhesion molecule, MAGE-A1, Mesothelin (MSLN), Murine CMV infected cells, MUC1, MUC16, NKG2D Ligands, NY-ESO-1, Oncofetal antigen, PSCA, ROR1, ROR2, Targeting via mAb IgE, TAG-72, VEGF-R2, Biotinylated molecules, CLDN18, B7-H3, GPC3, and CD147.

Introducing of nucleic acid molecules, e.g., heterologous or recombinant nucleic acid molecules, encoding a recombinant protein into a cell may be carried out using any of a number of methods known to the skilled persion. Such method may include the use of vectors, including viral vectors, including lentiviral and gammaretroviral systems. Exemplary methods include those for transfer of heterologous nucleic acid molecules encoding the recombinant proteins, including via transduction with a viral particle, e.g. retroviral vector particle or lentiviral vector particle.

After the introduction of the recombinant nucleic acid dislosed herein, the methods disclosed herein can further include one or more steps of incubating the cells after the introducing or contacting of the cells with the viral vector, such as incubating the one or more T cells under conditions for viral integration. Such conditions may comprise incubation at a temperature of at or about at or about 37°± 2° C. Said incubating of the one or more T cells after said introducing can be carried out for up to about 96 hours, up to about 72 hours, up to about 48 hours, up to about 24 hours, or up to about 18 hours subsequent to said introducing.

Introducing of a recombindant nucleic acid can also be carried out using a CRISPR-Cas9 combination. The CRISPR-Cas9 combination may comprise a guide RNA (gRNA), which may have a targeting domain that is complementary to a target site in the genome of the one or more T cells. In some cases, the CRISPR-Cas9 combination is a ribonucleoprotein (RNP) complex that includes the gRNA and a Cas9 protein. In some cases, the introduction of the recombinant nucleic acid is effected by introducing the RNP into the one or more T cells via electroporation.

The one or more isolated T cells (e.g., CD3+ T cells, CD4+ T cells, and/or CD8+ T cells) can generally be genetically engineered, transformed, or transduced after the cells have been isolated. In some cases, the recombinant nucleic acid molecule is introduced to the cells at, at about, or within 72 hours, 60 hours, 48 hours, 36 hours, 24 hours, 12 hours, 5 hours, 4 hours, or 2 hours, after their isolation. In some cases, the one or more isolated T cells may be cryopreserved and/or eryoprotected (e.g., frozen) and then thawed prior introducing recombinant nucleic acid molecule into the cells.

According to the methods of the present disclosure, the isolated one or more T cells and/or engineered one or more T cells may be incubated under cultivating conditions that promote proliferation and/or expansion to the cells. Such cultivating conditions may be designed to induce proliferation, expansion, activation, and/or survival of cells in the population. Such cultivating conditions can include one or more of particular media, temperature, oxygen content, carbon dioxide content, time, agents, e.g., nutrients, aminoacids, antibiotics, ions, and/or stimulatory factors, such as cytokines, ehemokines, antigens, binding partners, fusion proteins, recombinant soluble receptors, and any other agents designed to promote growth, division, and/or expansion of the cells. The cultivating is carried out for a time that is no more than 14 days, no more than 12 days, no more than 10 days, no more than 8 days, no more than 6 days, or no more than 5 days.

The methods of the present disclosure may also comprise a step of activating the one or more isolated T cells and/or the one or more engineered T cells. The activation step can be carried out after the isolation step and before the engineering step, such as before introducing the recombinant nucleic acid molecules into the cells. The activation step can alse be carried out after the engineering step, such as introducing the recombinant nucleic acid molecules into the cells. Generally, the activating step can be carried out by cultivating the one or more isolated T cells and/or the one or more engineered T cells under stimulatory conditions. The activation step may comprise contacting the one or more isolated T cells and/or the one or more engineered T cells with a stimulatory reagent, where the initiation of the activation preferably occurs when the cells are incubated or contacted with the stimulatory reagent. In some cases, the stimulating conditions or stimulatory reagents (e.g., oligomeric stimulatory reagents) include one or more stimulatory agent, which is capable of activating an intracellular signaling domain of a TCR complex. Generally, the stimulatory reagent may be any stimulatory reagent, including oligomeric stimulatory reagent disclosed herein, such as *supra* or *infra.* In some cases, a stimulatory reagent agent as contemplated herein can include, but is not limited to, RNA, DNA, proteins (e.g., enzymes), antigens, polyclonal antibodies, monoclonal antibodies, antibody fragments, carbohydrates, lipids lectins, or any other biomolecule with an affinity for a desired target. In some cases, the desired target is a T cell receptor and/or a component of a T cell receptor. In certain embodiments, the desired target is CD3. In certain embodiment, the desired target is a T cell costimulatory molecule, e.g., CD28, CD137 (4-l -BB), OX40, or ICOS.

The stimulatory reagent (e.g., oligomeric stimulatory reagent) may contain one or more stimulatory agents that bind to one or more of the following macromolecules on. a cell (e.g., a T cell): GD2, CD3, CD4, CDS, CDS, CD25, CD27, CD28, CD29, CD31, CD44, CD45RA, CD45RÖ, CD54 (ICAM-1), CD127, MHCI, MUCH, CTLA-4, ICOS, PD-l , 0X40, CD27L (CD70), 44BB (CD137), 4-1BBL, CD30L, LIGHT, IL-2R, IL- 12R , IL-1R, IL-15R; IFM-gammaR, TNF-alphaR, IL-4R, IL- 10R, CDI8/CD1 la (LFA- 1), CD62L (L-selectin), CD29/CD49d (VLA-4), Notch ligand (e.g. Delta-like 1/4, Jagged 1/2, etc,), CCR 1 , CCR2, CCR3, CCR4, CCR5, CCR7, and CXCR3 or fragment thereof including the corresponding ligands to these macromolecules or fragments thereof. In some cases, a stimulatory agent specifically binds to one or more of the following macromolecules on a cell (e.g. a T cell): CD28, CD62L, CCR7S CD27, CD127, CD3, CD4, CD8, CD45RA, and/or CD45RO. In some cases, the stimulatory agent is an antibody that binds to and/or recognizes one or more components of a T cell receptor, such as an anti-CD3 antibody. In cases, the stimulatory agent is an antibody that binds to and/or recognizes a costimulatory molecule, such as an anti-CD28 antibody. In some cases, the stimulatory reagent comprises an anti-CD28 antibody and an anti-CD3 antibody. For example, the stimulatory reagent may comprise a first stimulatory agent, which is an anti-CD3 Fab, for example as described herein, and a second stimulatory agent, which is an anti-CD28 Fab, for example as described herein. The stimulatory reagent can be an oligomeric stimulatory reagent as disclosed herein.

A T cell of the disclosure, including a plurality of T cells according to the disclosure, one or more T cells according to the disclosure, one or more isolated T cells according to the disclosure, one or more engineered T cells according ot the disclosure, etc., is preferably a human T cell. For example a plurality of T cells according to the disclosure is preferably a plurality of human T cells, one or more T cells according to the disclosure are preferably one or more human T cells, one or more isolated T cells according to the disclosure are preferably one or more isolated human T cells, and one or more engineered T cells according ot the disclosure are preferably one or more engineered human T cells.

The present invention also relates to a compositon comprising one or more T cells, including one or more isolated T cells and/or one or more engineered T cells, obtainable by the methods of the invention.

A composition of the disclosure may be a pharmaceutical composition. As such, the one or more T cells, including one or more isolated T cells and/or one or more engineered T cells can be formulated in a pharmaceutically acceptable buffer, which may, in some aspects, include a pharmaceutically acceptable carrier or excipient.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some aspects, the choice of carrier is determined in part by the particular cell and/or by the method of administration. Accordingly, there are a variety of suitable formulations. For example, the pharmaceutical can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic add and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (eg. Zn-protein complexes); and/or non-ionic surfactants such as polyethylene glycol (PEG).

In some cases, one or more buffering agents are included in the compositions of the disclosure. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrate pharmaceutical compositions are known. Exemplary methods are described in more detail in, for example. Remington: The Science and Practice of Pharmacy, Lippincott Williams & Wilkins; 21st ed. (May 1, 2005).

The formulation or composition can include an aqueous solution. The formulation or composition may also contain more than one active ingredient useful for the particular indication, disease, or condition being treated with the cells, preferably those with activities complementary to the cells, where the respective activities do not adversely affect one another. Such active ingredients are suitably present in combination in amounts that are effective for the purpose intended. Thus, in some embodiments, the pharmaceutical composition further includes other pharmaceutically active agents or drugs.

Compositions can be provided as sterile liquid preparations, e.g., isotonic aqueous solutions, suspensions, emulsions, dispersions, or viscous compositions, which may in some aspects be buffered to a selected pH. Liquid compositions can comprise carriers, which can be a solvent or dispersing medium containing, for example, water, saline, phosphate buffered saline, polyol (for example, glycerol, propylene glycol, liquid polyethylene glycol) and suitable mixtures thereof. Sterile injectable solutions can be prepared by incorporating the cells in a solvent, such as in admixture with a suitable carrier, diluent, or excipient such as sterile water, physiological saline, glucose, dextrose, or the like. The compositions can contain auxiliary substances such as wetting, dispersing, or emulsifying agents (e.g., methylcellulose), pH buffering agents, gelling or viscosity enhancing additives, preservatives, flavoring agents, and/or colors, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, and sorbic acid. Prolonged absorption of the injectable pharmaceutical form can be brought about by the use of agents delaying absorption, for example, aluminum monostearate and gelatin.

The composition can contain a cryopreservative. For example, the cells can be formulated with (with other words, the composition may comprise) a cyropreservative solution that contains 1.0% to 30% DMSO solution, such as a 5% to 20% DMSO solution or a 5% to 10% DMSO solution. The cryopreservation solution can be or contain, for example, PBS containing 20% DMSO and 8% human serum albumin (HSA), or other suitable cell freezing media. The cryopreservative solution can be or contain, for example, at least or about 7.5% DMSO. In some cases, the cells can be frozen, e.g., cryoprotected or cryopreserved, in media and/or solution with a final concentration of or of about 12.5%, 12.0%, 11.5%, 11.0%, 10.5%, 10.0%, 9.5%, 9. 0%, 8.5%, 8.0%, 7.5%, 7.0%, 6.5%, 6.0%, 5.5%, or 5.0% DMSO, or between 1% and 15%, between 6% and 12%, between 5% and 10%, or between 6% and 8% DMSO. For example, the cells can be frozen, e.g., cryoprotected or cryopreserved, in media and/or solution with a final concentration of or of about 5.0%, 4.5%, 4.0%, 3,5%, 3.0%, 2.5%, 2.0%, 1.5%, 1.25%, 1.0%, 0.75%, 0.5%, or 0.25% HSA, or between 0.1% and -5%, between 0.25% and 4%, between 0.5% and 2%, or between 1% and 2% HSA.

The present invention also relates to a method of treating a disease comprising administering to a subject a one or more T cells, including one or more isolated T cells and/or one or more engineered T cells, obtainable by any method of the disclosure or a composition comprising said one or more T cells. Said composition may be any composition comprising said one or more T cells disclosed herein. The subject is preferably in need thereof. The one or more T cells and/or the composition is preferably administered in an effective amount. The disease is preferably a cancer or tumor, an autoimmune disease, or an infectious disease. The method of treating may comprise, prior to administration to the subject, isolating the one or more T cells according to a method of the disclosure and/or engineering the one or more T cells according to a method of the dislcoures.

A "subject" is a vertebrate, preferably a mammal, more preferably a human. The term "mammal" is used herein to refer to any animal classified as a mammal, including, without limitation, humans, domestic and farm animals, and zoo, sports, or pet animals, such as mice, sheep, dogs, horses, cats, cows, rats, pigs, apes such as cynomolgus monkeys and etc., to name only a few illustrative examples. Preferably, the mammal herein is human.

An "effective amount" is an amount sufficient to effect beneficial or desired results. An effective amount can be administered in one or more administrations.

A "cancer" or "tumor" may be any cancer or tumor disclosed herein. Generally, the cancer or tumor can be selected from the group consisting of carcinoma, melanoma, blastoma, lymphoma and sarcoma. The cancer or tumor can be relapsed and/or refractory cancer or tumor.

Generally, the one or more T cells or the composition can be administered via any suitable route that is known to the skilled person. Administration can include parenteral administration. Parenteral administration can include local administration, such as intramuscular, intraperitoneal, subcutaneous, or intratumoral administration. Alternatively, parenteral administration can include systemic administration, in particular intravenous administration, such as via injection or infusion.

The present invention also relates to one or more T cells, including one or more isolated T cells and/or one or more engineered T cells, obtainable by any method of the disclosure, or a composition comprising said one or more T cells, such as a composition disclosed herein, for use in therapy. The use may be in a method of treatment disclosed herein, such as for the treatment of a cancer or tumor, an autoimmune disease or an infectious disease. The use may comprise, prior to administration to the subject, isolating the one or more T cells according to a method of the disclosure and/or engineering the one or more T cells according to a method of the dislcoures.

The present invention also relates to a use of one or more T cells, including one or more isolated T cells and/or one or more engineered T cells, obtainable by any method of the disclosure, or a composition comprising said one or more T cells, such as a composition disclosed herein, for the manufacture of a medicament. The medicament is preferably for any method of treatment disclosed herein, such as for the treatment of a cancer or tumor, an autoimmune disease or an infectious disease. The use may comprise, prior to administration to the subject, isolating the one or more T cells according to a method of the disclosure and/or engineering the one or more T cells according to a method of the dislcoures.

It must be noted that as used herein, the singular forms "a", "an" and "the" include plural references and vice versa unless the context clearly indicates otherwise.

Unless otherwise indicated, the term "at least" preceding a series of elements is to be understood to refer to every element in the series.

Those skilled in the art will recognize or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

Throughout this specification and the claims, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms. For example, when disclosure uses the term "comprising", the disclosure also encompasses the replacement of the term "comprising" with the terms "consisting essentially of" as well as "consisting of" and *vice versa.* E.g., the term "comprising" is meant to provide explicit support also for its replacement with "consisting essentially of" and/or "consisting of", the term "consisting essentially of" is meant to provide explicit support also for its replacement with "comprising" and/or "consisting of", and the term "consisting of" is meant to provide explicit support also for its replacement with "consisting essentially of" and "comprising". The possibility to replace terms with each other is not to be understood that these terms are synonymous. For the avoidance of doubt, the term "comprising", "consisting essentially of" or "consisting of" that is explicitly recited in the respective context is the preferred term, while its replacement with any one of the other two terms is less preferred.

The invention is further characterized by the following items.

Item 1. A method of isolating one or more T cells, the method comprising
(a) contacting a plurality of T cells with a selection agent that is specific for a receptor on one or more of the T cells,
   (i) wherein the selection agent is attached to a solid phase; or
   (ii) wherein the selection agent is not attached to a solid phase and the method further comprises attaching the selection agent to a solid phase; and
(b) incubating the one or more T cells at about 19 °C to about 42°C for about 1 h to about 24 h, thereby inducing downregulation of said receptor on the T cell, resulting in relase of one or more of the plurality of the T cells from the selection agent;
wherein steps (a) and (b) are conducted in absence of an oligomeric stimulatory reagent that comprises a specific binding partner for CD3 and/or a specific binding partner for CD28.

Item 2. The method of item 1, wherein the method further comprises
(c) collecting one or more of the plurality of T cells from the solid phase, thereby generating a composition comprising one or more isolated T cells,

Item 3. The method of item 2, wherein the one or more isolated T cells are unstimulated.

Item 4. The method of any one of the preceding items, wherein the solid phase is or comprises a chromatography matrix or a synthetic bead particle.

Item 5. The method of any one of the preceding items, wherein the synthetic bead particle is in solution.

Item 6. The method of any one of the preceding items, wherein steps a. and b. are conducted in absence of a T cell stimulatory agent, wherein the T cell stimulatory agent is preferably capable of delivering a stimulatory signal in the T cell, wherein the stimulatory signal is preferably through a TCR/CD3 complex in a T cell, a CD3-containing complex in a T cell, and/or an ITAM-containing molecule in a T cell.

Item 7. The method of any one of the preceding items, wherein steps (a) and (b) are conducted in absence of a T cell stimulatory agent that is able to cross-link TCR/CD3 complex and T cell co-stimulatory molecule(s) simultaneously or in absence of an oligomeric binding agents specific for one or more targets selected from CD3, TCR, CD28, CD90 (Thy-1), CD95 (Apo-/Fas), CD137 (4-1BB), CD154 (CD40L), ICOS, CD27, OX40 or HYEM.

Item 8. The method of item 7, wherein the T cell stimulatory agent is antibody-based.

Item 9. The method of item 7 or 8, wherein the T cell stimulatory agent is Fab-based.

Item 10. The method of any one of the preceding items, wherein steps (a) and (b) are conducted without addition of an elution reagent, wherein the elution reagent is preferably selected from the group consisting of biotin and D-biotin.

Item 11. The method of any one of the preceding items, wherein steps (a) and (b) are conducted without disrupting the attachment of the specific binding agent to the solid phase.

Item 12. The method of any one of the preceding items, wherein the incubation in step (b) is carried out at about 24°C to about 40°C, preferably about 30°C to about 39°C, preferably about 35°C to about 38°C, preferably about 37°C.

Item 13. The method of any one of the preceding items, wherein the incubation in step (b) is carried out for about 1 h to about 18 h, preferably about 1 h to about 12 h, preferably about 2 h to about 8 h, preferably about 2 h to about 6 h, preferably about 2 h to about 4 h.

Item 14. The method of any one of the preceding items, wherein the plurality of T cells is from a sample that is or comprises a whole blood sample, a buffy coat sample, a peripheral blood mononuclear cells (PBMC) sample, an unfractionated T cell sample, a lymphocyte sample, a white blood cell sample, an apheresis product, or a leukapheresis product.

Item 15. The method of item 14, wherein the sample is an apheresis or leukapheresis product.

Item 16. The method of item 14 or 15, wherein the apheresis or leukapheresis product has been previously cryopreserved.

Item 17. The method of any one of the preceding items, wherein the receptor on the one or more of the T cells is selected from the group consisting of:
(i) a receptor that is a T cell coreceptor;
(ii) a receptor that is or comprises a member of a T cell antigen receptor complex;
(iii) a receptor that is or comprises a TCR alpha chain and/or beta chain;
(iv) a receptor that is or comprises CD3;
(v) a receptor that is or comprises a CD3 chain;
(vi) a receptor that is or comprises a CD3 gamma chain;
(vii) a receptor that is or comprises a CD3 delta chain;
(viii) a receptor that is or comprises a CD3 epsilon chain;
(ix) a receptor that is or comprises a CD3 zeta chain;
(x) a receptor that is or comprises CD8;
(xi) a receptor that is or comprises CD4;
(xii) a receptor that is or comprises CD45RA;
(xiii) a receptor that is or comprises CD27;
(xiv) a receptor that is or comprises CD28;
(xv) a receptor that is or comprises CD137 (4-1BB);
(xvi) a receptor that is or comprises CCR7;
(xvii) a receptor that is or comprises CD62L; and/or
(xviii) a receptor that is or comprises CD25.

Item 18. The method of any one of the preceding items, wherein the receptor on the one or more of the T cells is selected from the group consisting of CD3, CD4, and CD8.

Item 19. The method of any one of the preceding items, wherein the receptor on the one or more of the T cells is CD3.

Item 20. The method of any one of the preceding items, wherein the specific binding between the selection agent and the receptor on the one or more of the T cells does not induce a proliferative signal, to the one or more of the T cells.

Item 21. The method of any one of the preceding items, wherein the selection agent comprises at least an antigen binding fragment of an antibody that specifically binds the receptor on the T cell.

Item 22. The method of any one of the preceding items, wherein the selection agent comprises a mono-, bi-, or multivalent antibody, antibody construct, or antibody fragment that binds to the receptor on the T cell, wherein said receptor on the T cell is preferably CD3, CD8, or CD4.

Item 23. The method of any one of the preceding items, wherein the selection agent is or comprises a Fab that binds to the receptor on the T cell, preferably an anti-CD3 Fab, an anti-CD8 Fab, or an anti-CD4 Fab.

Item 24. The method of any one of the preceding items, wherein the selection agent is an anti-CD3 Fab.

Item 25. The method of any one of the preceding items, wherein the selection agent is directly or indirectly bound to the solid phase.

Item 26. The method of any one of the preceding items, wherein the selection agent is bound indirectly to the solid phase through a selection reagent to which the selection agent binds reversibly or irreversibly.

Item 27. The method of any one of the preceding items, wherein the selection reagent is or comprises streptavidin, avidin, a mutein of streptavidin that irreversibly or reversibly binds biotin, a biotin analog or a biologically active fragment thereof; a mutein of avidin or streptavidin that irreversibly or reversibly binds a streptavidin-binding peptide; a reagent that comprises at least two chelating groups K, wherein the at least two chelating groups are capable of binding to a transition metal ion; an agent capable of binding to an oligohistidine affinity tag; an agent capable of binding to a glutathione-S-transferase; calmodulin or an analog thereof; an agent capable of binding to calmodulin binding peptide (CBP); an agent capable of binding to a FLAG-peptide; an agent capable of binding to an HA-tag; an agent capable of binding to maltose binding protein (MBP); an agent capable of binding to an HSV epitope; an agent capable of binding to a myc epitope; or an agent capable of binding to a biotinylated carrier protein.

Item 28. The method of any one of the preceding items, wherein the selection reagent is or comprises a streptavidin mutein or an avidin mutein that reversibly binds to biotin or a biologically active fragment.

Item 29. The method of item 27, wherein the streptavidin or streptavidin mutein molecules reversibly bind to or are capable of reversibly binding to biotin, a biotin analog or a streptavidin-binding peptide.

Item 30. The method of any one of items 1-25, wherein the selection agent is covalently bound to the solid phase.

Item 31. The method of any one of items 2-30, wherein said collecting is by filtration to separate the one or more of the plurality of T cells from the solid phase.

Item 32. The method of any one of items 2-31, wherein said collecting is by gravity flow and preferably comprises adding media to the solid phase, the media not comprising a competition agent or free binding agent to elute the one or more of the plurality of T cells from the solid phase.

Item 33. The method of any one of items 2-32, wherein said composition comprising the isolated one or more T cells does not comprise a competition agent or free binding agent.

Item 34. The method of item 32 or 33, wherein said competition agent or free binding agent is or comprises biotin or a biotin analog, optionally wherein the biotin analog is D-biotin.

Item 35. The method of any one of the preceding items, wherein the solid phase is or comprises a chromatography matrix and/or a synthetic bead particle.

Item 36. The method of any one of the preceding items, further comprising introducing a recombinant nucleic acid molecule into the isolated one or more T cells, wherein the nucleic acid molecule encodes a recombinant protein, thereby producing one or more engineered T cells.

Item 37. The method of item 36, wherein the recombinant protein is an antigen receptor.

Item 38. The method of item 36 or 37, wherein the recombinant protein is a chimeric antigen receptor (CAR) or T cell receptor (TCR).

Item 39. The method of item 38, wherein the chimeric antigen receptor CAR comprises an extracellular antigen-recognition domain that specifically binds to a target antigen and an intracellular signaling domain comprising an ITAM.

Item 40. The method of item 39, wherein the intracellular signaling domain comprises an intracellular domain of a CD3-zeta (CD3ζ chain).

Item 41. The method of item 39 or 40, wherein the CAR further comprises a transmembrane domain linking the extracellular domain and the intracellular signaling domain.

Item 42. The method of item 41, wherein the transmembrane domain comprises a transmembrane portion of CD28.

Item 43. The method of any of items 39-42, wherein the intracellular signaling domain further comprises an intracellular signaling domain of a T cell costimulatory molecule.

Item 44. The method of item 43, wherein the T cell costimulatory molecule is selected from the group consisting of CD28 and 4-1BB.

Item 45. The method of any of items 36-44, wherein the introduction of the recombinant nucleic acid is achieved by transduction with a viral particle.

Item 46. The method of item 45, wherein the viral particle is a retroviral vector particle.

Item 47. The method of item 45 or 46, wherein the viral particle is a lentiviral vector particle.

Item 48. The method of any one of items 45-47, further comprising incubating the one or more T cells after the introducing under conditions for viral integration, optionally at a temperature of at or about 37°± 2° C.

Item 49. The method of item 48, wherein said incubating of the one or more T cells after said introducing is carried out for up to about 96 hours, up to about 72 hours, up to about 48 hours, up to about 24 hours, or up to about 18 hours subsequent to said introducing.

Item 50. The method of any of items 36-44, wherein the introduction of the recombinant nucleic acid is effected by a CRISPR-Cas9 combination.

Item 51. The method of item 50, wherein the CRISPR-Cas9 combination comprises a guide RNA (gRNA) and the gRNA has a targeting domain that is complementary to a target site in genome of the one or more T cells.

Item 52. The method of item 50 or 51, wherein the CRISPR-Cas9 combination is a ribonucleoprotein (RNP) complex comprising the gRNA and a Cas9 protein.

Item 53. The method of item 52, wherein the introduction of the recombinant nucleic acid is effected by the RNP introduced into the one or more T cells via electroporation.

Item 54. The method of any one of the preceding items, further comprising cultivating the one or more isolated T cells and/or the one or more engineered T cells, under conditions to expand the T cells.

Item 55. The method of item 54, wherein the cultivating is carried out for a time that is no more than 14 days, no more than 12 days, no more than 10 days, no more than 8 days, no more than 6 days, or no more than 5 days.

Item 56. The method of any one of the preceding items, further comprising activating the one or more T cells comprising contacting the isolated one or more isolated T cells and/or the one or more engineered T cells with an oligomeric stimulatory reagent capable of delivering a stimulatory signal in T cells.

Item 57. The method of item 56, wherein the oligomeric stimulatory reagent is the olimogeric stimulatory reagent as defined in any one of the preceding items.

Item 58. The method of any one of the preceding items, wherein said one or more T cells are human T cells.

Item 59. A composition comprising one or more T cells obtainable by the method of any one of items 1-58.

Item 60. The composition of item 59, wherein the composition is a pharmaceutical composition

Item 61. A method of treating a disease comprising administering to a subject one or more T cells or a composition comprising said one or more T cells obtainable by the method of any one of items 1-58 or the composition of item 59 or 60.

Item 62. The method of item 61, wherein the method comprises isolating said one or more T cells according to the method of any one of items 1-58 prior to said administering.

Item 63. The method of item 61 or 62, wherein the disease is cancer or an autoimmune disease.

Item 64. The method of any one of items 61-63, wherein the subject is human.

Item 65. One or more T cells obtainable by the method of any one of items 1-58 or a composition of item 58 or 59 for use in therapy.

Item 66. The one or more T cells or the composition for the use of item 65, wherein the use is in a method of any one of items 61-64.

Item 67. A use of one or more T cells obtainable by the method of any one of items 1-58 or a composition of item 58 or 59 for the manufacture of a medicament.

Item 68. The use of item 64, wherein the medicament is for use is in a method of any one of items 61-64.

### EXAMPLES

### Example 1

Example 1 describes selection of CD3+ target cells from PBMC fraction of a buffy coat (Figure 1). First total PBMCs were passed trough an anti-CD3 selection resin loaded in a fixed container so that target cells can be captured. Subsequently, selection resin containing captured target cells was transferred to an incubator and incubated for 6 hours at 37C. Afterward, target cells were released from selection matrix due to TCR/CD3 complex downregulation and removed from the container via liquid exchange. Collected cells displayed high purity as indicated by low content of CD19+, CD56+, CD14+ cells (Figure 1A) as well as clear TCR/CD3 complex downregulation as measured by CD3 expression (Figure 1D). Noteworthy, selected cells remained unstimulated as depicted by no change in CD69 early activation marker expression before and after selection (Figure 1B). This selection procedure resulted in depletion, purity and yield within anticipated ranges established using other cell selection methodologies (Figure 1C).

### Example 2

Example 2 describes cell selection in a presence or absence of soluble cell stimulation agent when the selection material was based on anti-CD3 coated synthetic bead particles in solution (Figure 2). PBMC fraction of a buffy coat was mixed with anti-CD3 selection beads on a filter membrane. Traget cells captured on anti-CD3 selection beads were transferred into incubator and incubated for 6 hours at 37C. Subsequently, cells were released from the bead particles via TCR/CD3 complex downregulation and collected in a vessel below the filter. Presence or absence of stimulatiory agent did not alter selection performance as indicated by cell composition after selection (Figure 2A), purity and yield (Figure 2B), and TCR/CD3 complex downregulation (Figure 2C).

### Example 3

Example 3 descirbes cell selection using ssame methodology as Example 1 but using anti-CD4 (Figure 3A) or anti-CD8 cell selection resin (Figure 3B). Noteworthy, CD4 and CD8 receptors follow similar downregulation principle to TCR/CD3 complex.

Embodiments illustratively described herein may suitably be practiced in the absence of any element or elements, limitation or limitations, not specifically disclosed herein. Thus, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present embodiments have been specifically disclosed by preferred embodiments and optional features, modification and variations thereof may be resorted to by those skilled in the art, and that such modifications and variations are considered to be within the scope of this invention. Each of the narrower species and subgeneric groupings falling within the generic disclosure also forms part of the invention. This includes the generic description of the invention with a proviso or negative limitation removing any subject matter from the genus, regardless of whether or not the excised material is specifically recited herein. In addition, where features are described in terms of Markush groups, those skilled in the art will recognize that the disclosure is also thereby described in terms of any individual member or subgroup of members of the Markush group.

Equivalents: Those skilled in the art will recognize or be able to ascertain using no more than routine experimentation, many equivalents to the specific embodiments of the invention described herein. Such equivalents are intended to be encompassed by the following claims.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications cited throughout the text of this specification (including all patents, patent applications, scientific publications, manufacturer's specifications, instructions, etc.) are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

Further embodiments will become apparent from the following claims.

## Claims

1. A method of isolating one or more T cells, the method comprising
(a) contacting a plurality of T cells with a selection agent that is specific for a receptor on one or more of the T cells,
(i) wherein the selection agent is attached to a solid phase; or
(ii) wherein the selection agent is not attached to a solid phase and the method further comprises attaching the selection agent to a solid phase; and
(b) incubating the one or more T cells at about 19 °C to about 42°C for about 1 h to about 24 h, thereby inducing downregulation of said receptor on the T cell, resulting in relase of one or more of the plurality of the T cells from the selection agent;
wherein steps (a) and (b) are conducted in absence of an oligomeric stimulatory reagent that comprises a specific binding partner for CD3 and/or a specific binding partner for CD28.

2. The method of claim 1, wherein the method further comprises
(c) collecting one or more of the plurality of T cells from the solid phase, thereby generating a composition comprising one or more isolated T cells,

3. The method of claim 2, wherein the one or more isolated T cells are unstimulated.

4. The method of any one of the preceding claims, wherein steps (a) and (b) are conducted in absence of a T cell stimulatory agent that is able to cross-link TCR/CD3 complex and T cell co-stimulatory molecule(s) simultaneously or in absence of an oligomeric binding agents specific for one or more targets selected from CD3, TCR, CD28, CD90 (Thy-1), CD95 (Apo-/Fas), CD137 (4-1BB), CD154 (CD40L), ICOS, CD27, OX40 or HYEM.

5. The method of any one of the preceding claims, wherein steps (a) and (b) are conducted without disrupting the attachment of the specific binding agent to the solid phase.

6. The method of any one of the preceding claims, wherein the incubation in step (b) is carried out at about 24°C to about 40°C, preferably about 30°C to about 39°C, preferably about 35°C to about 38°C, preferably about 37°C, and/or wherein the incubation in step (b) is carried out for about 1 h to about 18 h, preferably about 1 h to about 12 h, preferably about 2 h to about 8 h, preferably about 2 h to about 6 h, preferably about 2 h to about 4 h.

7. The method of any one of the preceding claims, wherein the receptor on the one or more of the T cells is selected from the group consisting of:
(i) a receptor that is a T cell coreceptor;
(ii) a receptor that is or comprises a member of a T cell antigen receptor complex;
(iii) a receptor that is or comprises a TCR alpha chain and/or beta chain;
(iv) a receptor that is or comprises CD3;
(v) a receptor that is or comprises a CD3 chain;
(vi) a receptor that is or comprises a CD3 gamma chain;
(vii) a receptor that is or comprises a CD3 delta chain;
(viii) a receptor that is or comprises a CD3 epsilon chain;
(ix) a receptor that is or comprises a CD3 zeta chain;
(x) a receptor that is or comprises CD8;
(xi) a receptor that is or comprises CD4;
(xii) a receptor that is or comprises CD45RA;
(xiii) a receptor that is or comprises CD27;
(xiv) a receptor that is or comprises CD28;
(xv) a receptor that is or comprises CD137 (4-1BB);
(xvi) a receptor that is or comprises CCR7;
(xvii) a receptor that is or comprises CD62L; and/or
(xviii) a receptor that is or comprises CD25;
wherein the receptor on the one or more of the T cells is preferably selected from the group consisting of CD3, CD4, and CD8.

8. The method of any one of claims 2-7, wherein said collecting is
(a) by filtration to separate the one or more of the plurality of T cells from the solid phase; or
(b) by gravity flow and preferably comprises adding media to the solid phase, the media not comprising a competition agent or free binding agent to elute the one or more of the plurality of T cells from the solid phase.

9. The method of any one of claims 2-8, wherein said composition comprising the isolated one or more T cells does not comprise a competition agent or free binding agent.

10. The method of any one of the preceding claims, wherein the solid phase is or comprises a chromatography matrix and/or a synthetic bead particle.

11. The method of any one of the preceding claims, further comprising introducing a recombinant nucleic acid molecule into the isolated one or more T cells, wherein the nucleic acid molecule encodes a recombinant protein, thereby producing one or more engineered T cells, wherein the recombinant protein is preferably an antigen receptor, preferably a chimeric antigen receptor (CAR) or T cell receptor (TCR).

12. The method of any one of the preceding claims, further comprising cultivating the one or more isolated T cells and/or the one or more engineered T cells, under conditions to expand the T cells.

13. The method of any one of the preceding claims, further comprising activating the one or more T cells comprising contacting the isolated one or more isolated T cells and/or the one or more engineered T cells with an oligomeric stimulatory reagent capable of delivering a stimulatory signal in T cells.

14. A composition comprising one or more T cells obtainable by the method of any one of claims 1-13, wherein the composition is preferably a pharmaceutical composition.

15. One or more T cells obtainable by the method of any one of claims 1-58 or a composition of claim 58 or 59 for use in therapy, preferably for use in treating cancer or an autoimmune disease.
